# EUROPEAN PATENT APPLICATION

(11) **EP 4 661 600 A1**
(43) Date of publication of application: **10.12.2025**
(21) Application number: 23919814.6
(22) Date of filing: 02.08.2023
(51) Int. Cl.: H05H 13/00, H05H 13/04, A61N 5/10

(54) **CIRCULAR ACCELERATOR AND PARTICLE BEAM THERAPY SYSTEM HAVING CIRCULAR ACCELERATOR**

(30) Priority: 02.02.2023 JP 2023014926
(71) Applicant: HITACHI HIGH-TECH CORPORATION, Tokyo 105-6409 (JP)
(72) Inventor: MINAKAWA, Shunsuke, Tokyo 100-8280 (JP); HAE, Takamitsu, Tokyo 100-8280 (JP)
(74) Representative: MERH-IP Matias Erny Reichl Hoffmann Patentanwälte PartG mbB
(86) International application number: PCT/JP2023/028342
(87) International publication number: WO 2024/161678

(57) **Abstract**

To provide a circular accelerator capable of detecting the position of a circulating charged particle beam and a particle beam therapy system including the circular accelerator. A circular accelerator (39) configured to apply a first radio frequency to a circling charged particle beam to accelerate the charged particle beam, the circular accelerator including: an electrode (12), (13) configured to apply the first radio frequency to the charged particle beam; and a beam position monitor (16) provided in the electrode and configured to detect a position of the charged particle beam.

## Description

### Technical Field

The present invention relates to a circular accelerator and a particle beam therapy system including the circular accelerator.

### Background Art

A synchrocyclotron and an eccentric orbital accelerator described in PTL 1 are known as a circular accelerator of a type in which a main magnetic field strength is temporally constant and a frequency of an acceleration radio frequency is temporally modulated. In these circular accelerators, it is relatively easy to increase a magnetic field by using a superconducting coil for generating a main magnetic field, and thus the accelerator can be downsized. In addition, by downsizing the accelerator, the cost of the accelerator can be reduced. Therefore, these circular accelerators are particularly applied to a particle beam therapy system.

In a synchrocyclotron or an eccentric orbital accelerator, a position of a charged particle beam is grasped using a beam position monitor in order to grasp an orbit of the charged particle beam or specify a cause when acceleration fails.

As a beam position monitor, there is an electrostatic beam position monitor using a triangular plate electrode (PTL 2) . The beam position monitor is configured by a right-side electrode and a left-side electrode each having a triangular shape in plan view. The right-side electrode and the left-side electrode are formed in a lateral U-shape. The right-side electrode and the left-side electrode are attached in a vacuum container by way of an insulation member. The electrode plate constitutes a capacitance electrode. When the charged particle beam passes through between the right-side electrode and the left-side electrode, an RF voltage is generated between these electrodes. The RF voltage is output to a signal processing system via a signal terminal for the right-side electrode and a signal terminal for the left-side electrode.

PTL 3 describes an accelerator that accelerates a beam using a static magnetic field and a frequency-modulated radio frequency acceleration electric field. This accelerator extracts a beam by applying an electric field to a radio frequency kicker using a resonance phenomenon of betatron oscillation. Furthermore, PTL 3 describes that the intensity of the beam extracted from the circular accelerator can be controlled by controlling any of the strength of the voltage applied to the radio frequency kicker, the amplitude, the phase, and the frequency of the radio frequency.

### Citation List

### Patent Literature

PTL 1: JA 2019-133745 A
PTL 2: JA 2001-21698 A
PTL 3: JA 2019-158109 A

### Summary of Invention

### Technical Problem

In the synchrocyclotron or the eccentric orbital accelerator described in PTLs 1 and 3, the charged particle beam has a plurality of orbits having different radii of curvature according to energy. Therefore, in these accelerators, it is necessary to extract charged particle beams in different orbits in order to make the extracted energy vary. In order to extract the charged particle beam, the RF electric field of the radio frequency kicker is adjusted to the betatron oscillation frequency of the charged particle beam, and the charged particle beam is moved out of the orbit using the resonance phenomenon. In order to efficiently extract the charged particle beam by the RF electric field of the kicker, it is important to synchronize the RF electric field with the betatron oscillation frequency. On the other hand, PTL 1 and PTL 2 do not disclose means for acquiring the betatron oscillation frequency.

In order to acquire the betatron oscillation, a beam position monitor for measuring the position of the beam is required. The beam position monitor is configured such that electrode plates having slits face each other. When the beam passes through between the electrode plates, the charge amount of the electrode plates changes. The position of the beam can be acquired by signal processing the change in the charge amount as the change in the voltage value.

The beam position monitor described in PTL 2 can be applied only to that in which the orbits of the charged particle beams are the same, and cannot detect the positions of beams of a plurality of orbits having different radii of curvature.

The present invention has been made in view of the above problems, and an object of the present invention is to provide a circular accelerator capable of detecting the position of a circulating charged particle beam and a particle beam therapy system including the circular accelerator.

### Solution to Problem

In order to solve the above problems, a circular accelerator according to the present invention is a circular accelerator that applies a first radio frequency voltage to a circulating charged particle beam to accelerate the charged particle beam, and includes an electrode that applies a first radio frequency to the charged particle beam, and a position monitor that is provided in the electrode and detects a position of the charged particle beam.

### Advantageous Effects of Invention

According to the present invention, the position of the charged particle beam can be detected by a beam position monitor provided in an electrode that applies a radio frequency to the charged particle beam to accelerate the charged particle beam.

### Brief Description of Drawings

[FIG. 1] FIG. 1 is a perspective view illustrating an outer appearance of a circular accelerator according to the present embodiment.
[FIG. 2] FIG. 2 is a lateral cross-sectional view of the circular accelerator.
[FIG. 3] FIG. 3 is a cross-sectional view as viewed from III-III direction in FIG. 2.
[FIG. 4] FIG. 4 is a diagram illustrating a beam orbit for each energy.
[FIG. 5] FIG. 5 is a diagram illustrating a motion pattern of the circular accelerator.
[FIG. 6] FIG. 6 is a cross-sectional view illustrating an accelerating cavity and a rotating capacitor.
[FIG. 7] FIG. 7 is a cross-sectional view illustrating the accelerating cavity and a beam position monitor.
[FIG. 8] FIG. 8 is a schematic diagram of a beam position monitor using a triangular plate electrode.
[FIG. 9] FIG. 9 is a plan view of the beam position monitor.
[FIG. 10] FIG. 10 is a plan view illustrating a modified example of the beam position monitor.
[FIG. 11] FIG. 11 is an explanatory diagram illustrating a state in which the charged particle beam passes through the beam position monitor as viewed from XI-XI direction in FIG. 9.
[FIG. 12] FIG. 12 corresponds to FIG. 11, and is a time chart illustrating a state of a signal output from the beam position monitor according to a position at which the charged particle beam passes through the beam position monitor.
[FIG. 13] FIG. 13 illustrates an example of an operation flow of the circular accelerator when the beam position monitor measures the beam position.
[FIG. 14] FIG. 14 is a diagram illustrating an example of data when the beam position monitor acquires a betatron oscillation frequency of a charged particle beam.
[FIG. 15] FIG. 15 is a diagram illustrating an example of a flow of calculating the betatron oscillation frequency of the charged particle beam from a measured beam position.
[FIG. 16] FIG. 16 is an explanatory diagram illustrating a configuration in which the beam position monitor is provided in a dee electrode.
[FIG. 17] FIG. 17 is a diagram illustrating a configuration of a particle beam therapy system using a circular accelerator having a beam position monitor according to a second example.

### Description of Embodiments

Hereinafter, embodiments of the present invention will be described with reference to the drawings. In the present embodiment, as will be described later, the position of the circulating charged particle beam is measured during operation of the circular accelerator. In the present embodiment, a charged particle beam circulating in orbits having different radii of curvature according to energy is measured nondestructively during operation of the circular accelerator. Therefore, in the present embodiment, the beam position monitor is provided at a position that is not affected by the acceleration electric field, and the betatron oscillation frequency calculated from the detection signal of the beam position monitor is synchronized with the radio frequency electric field of the kicker. As an example, in the present embodiment, a beam position monitor is provided at a predetermined position in the dee electrode that accelerates the charged particle beam.

The circular accelerator of the present embodiment can be expressed as, for example, "a circular accelerator that applies radio frequency to a circulating charged particle beam to accelerate the charged particle beam, the circular accelerator including a beam position monitor that measures the circulating charged particle beam, wherein the beam position monitor is disposed in an electrode to which the radio frequency is applied". As a result, according to the present embodiment, in a circular accelerator such as a synchrocyclotron or an eccentric orbital accelerator, beam positions of a plurality of orbits can be measured without stopping the operation of the circular accelerator. Therefore, the particle beam therapy system including the circular accelerator according to the present embodiment can increase the throughput of the patient to be treated. Hereinafter, the charged particle beam may be abbreviated as a beam.

Note that "first radio frequency" can also be rephrased as a "first radio frequency voltage", and "second radio frequency" can also be rephrased as a "second radio frequency voltage".

### First Example

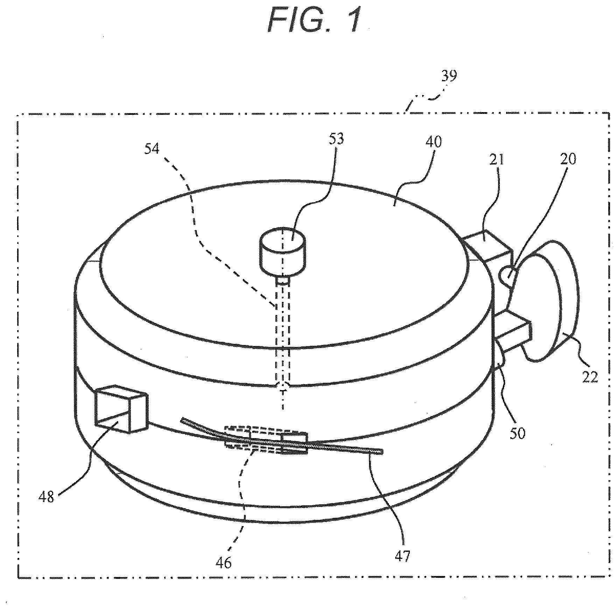

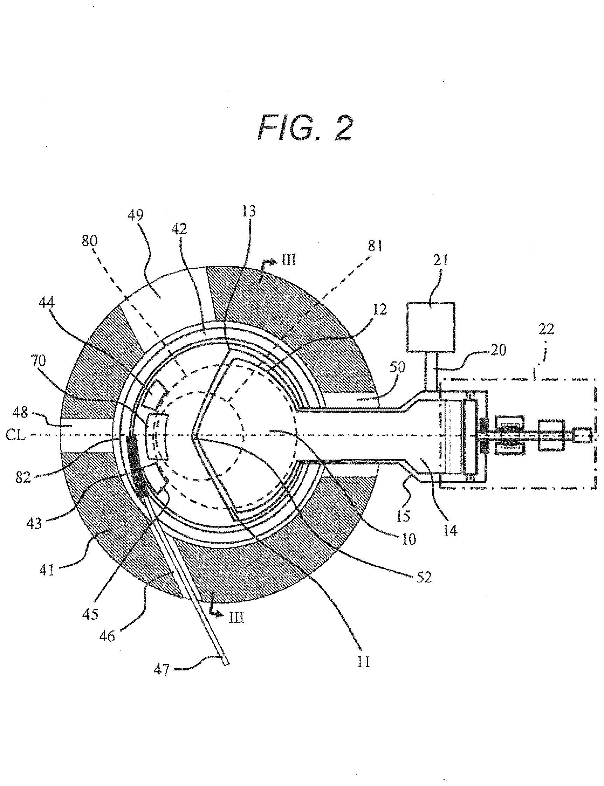

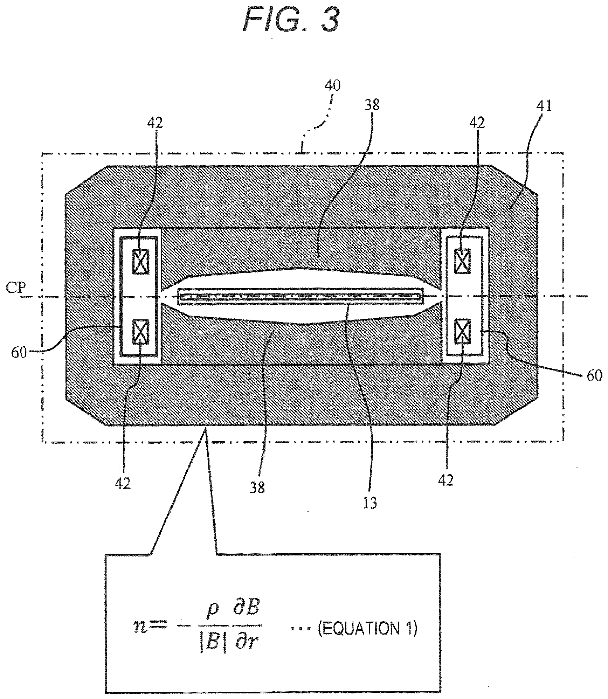

A first example will be described with reference to FIGS. 1 to 15. A configuration example of the circular accelerator 39 will be described with reference to Figs. 1 to 3. FIG. 1 is a perspective view illustrating an outer appearance of the circular accelerator 39 according to the present embodiment. FIG. 2 is a cross-sectional view illustrating a transverse section (center plane) of the circular accelerator 39. FIG. 3 is a cross-sectional view as viewed from III-III direction in FIG. 2. FIG. 3 illustrates a longitudinal section of the circular accelerator 39.

The circular accelerator 39 is a device that accelerates a beam by a radio frequency electric field that is frequency-modulated in a main magnetic field having a temporally constant strength. Here, as an example, a circular accelerator that accelerates a proton beam to 235 MeV will be described, but the circular accelerator 39 may be a device that accelerates a heavy particle beam such as helium or carbon.

The circular accelerator 39 is an eccentric orbital accelerator in which a main magnetic field is formed so as to eccentric the beam orbit toward the entrance of beam extraction path 82. The circular accelerator 39 can extract beam energy while arbitrarily varying between 70 MeV and 235 MeV.

As illustrated in FIGS. 1 and 3, the outer shell of the circular accelerator 39 is formed by a main electromagnet 40 that can be divided in the vertical direction. An acceleration region is formed on the center plane CP in the main electromagnet 40, and the acceleration region is vacuum drawn.

Hereinafter, an orbit through which the beam passes until the energy of the beam reaches 235 MeV of the maximum energy after the beam starts to be accelerated within the acceleration region is referred to as a closed orbit. Among the closed orbits, an orbit through which a beam in which an energy is a maximum energy of 235 MeV passes is referred to as a maximum energy orbit 80 (see FIG. 2). An orbit through which a beam in which an energy is 70 MeV passes is referred to as a minimum extraction energy orbit 81. A surface on which the closed orbit draws a spiral is referred to as an orbital surface or an orbital plane. A two-dimensional polar coordinate system of an orbital surface having the center of the acceleration region as an origin is defined, and an axis in a radially outer side direction from the center is referred to as an r axis.

As illustrated in FIG. 3, the main electromagnet 40 includes a main magnetic pole 38, a yoke 41, and a main coil 42. The outer appearance of the main electromagnet 40 is formed by the yoke 41. A substantially cylindrical region is formed inside the yoke 41. The main coil 42 is an annular superconducting coil and is installed along the inner wall of the yoke 41. A cryostat 60 is installed at the periphery of the main coil 42, and the main coil 42 is cooled by the cryostat 60. On the inner peripheral side of the main coil 42, a main magnetic pole 38 is installed to vertically face each other. The main magnetic pole 38 is excited by flowing a current to the main coil 42. The magnetic field in the vertical direction formed by the main magnetic pole 38 is referred to as a main magnetic field. The main magnetic field is used for forming an eccentric orbit. The acceleration region is a region for accelerating the beam in the main magnetic field.

As illustrated in FIG. 2, a plurality of through holes are formed in the yoke 41. For example, a beam through hole 46, a coil through hole 48, a vacuum drawing through hole 49, and a radio frequency system through hole 50 are formed in the yoke 41. The beam through hole 46 is a through hole for extracting an accelerated beam. The coil through hole 48 is a through hole for drawing out various coil conductors installed in the yoke 41 to the outside. The vacuum drawing through hole 49 is a through hole for vacuum drawing the acceleration region. The radio frequency system through hole 50 is a through hole for the accelerating hollow 10, and is provided on a connecting surface of the upper and lower magnetic poles.

As illustrated in FIG. 1, an ion source 53 is installed at the upper part of the main electromagnet 40. The ion source 53 generates a beam of ions (charged particle beam) injected on the main electromagnet 40. The beam generated by the ion source 53 passes through the low energy beam transport system 54, passes through an ion injection portion 52 (see FIG. 2), and is injected on an acceleration region inside the main electromagnet 40. As the ion source 53, an electron cyclotron resonance (ECR) ion source or the like can be applied. Note that the ion source 53 may be disposed in a vacuum drawn acceleration region inside the main electromagnet 40, in which case, a Penning or Phillips Ionization Gauge (PIG) type ion source or the like is suitable.

As illustrated in FIG. 2, the ion injection portion 52 is arranged closer to the entrance of beam extraction path 82 side than the machine center of the acceleration region on the center line CL. A beam of charged particles generated by the ion source 53 passes through the low energy beam transport system 54, passes through the ion injection portion 52, and is injected on an acceleration region inside the main electromagnet 40 by an inflector electrode (not illustrated) or the like. The injected beam is accelerated by a radio frequency electric field and circulates in the main magnetic field while increasing energy. As the beam is accelerated, the radius of curvature of its orbit increases. The beam draws a spiral orbit from the center of the acceleration region toward the outer side. Note that the radio frequency for accelerating the beam corresponds to an example of a "first radio frequency electric field".

The accelerating hollow 10 is a A/2 resonance cavity and includes a dee electrode 12, a dummy dee electrode 13, an inner conductor 14, an outer conductor 15, and a rotating capacitor 22. The dee electrode 12 is a hollow electrode through which the beam passes, and is electrically connected to the inner conductor 14. The dummy dee electrode 13 is an electrode having an earth potential, and is electrically connected to the outer conductor 15 enclosing the inner conductor 14. An acceleration gap 11 is formed between the dee electrode 12 and the dummy dee electrode 13. A radio frequency electric field is formed in the acceleration gap 11.

Here, the dee electrode is defined as a hollow semicylindrical electrode provided in a vacuum drawn space in the main magnet 38.

The radio frequency power to the accelerating hollow 10 is supplied by the radio frequency power supply 21 via an input coupler 20. The input coupler 20 is coupled with the accelerating hollow 10 in either electrostatic coupling or magnetic coupling method. As a result, a radio frequency electric field by a radio frequency acceleration voltage for accelerating the beam and a radio frequency acceleration voltage is generated in the acceleration gap 11.

The rotating capacitor 22 is a device that modulates the resonance frequency of the accelerating hollow 10. A frequency-modulated pattern can be formed by changing the resonance frequency of the accelerating hollow 10 by temporally varying the capacitance of the rotating capacitor 22. An acceleration voltage frequency-modulated by the rotating capacitor 22 is generated in the acceleration gap 11 between the dee electrode 12 and the dummy dee electrode 13. The acceleration gap 11 illustrated in FIG. 2 is an acceleration gap having a harmonic number of 1, that is, an acceleration gap in which the circulating frequency and the acceleration frequency are the same, and is formed according to the beam orbit shape.

The radio frequency power supply 21 supplies radio frequency power having a frequency following the change in resonance frequency of the accelerating hollow 10 by either a self-excited or an other-excited method.

Hereinafter, the main magnetic field that realizes the eccentric orbit will be described. The main magnetic field may be a magnetic field of a type in which the main magnetic field strength is constant in the circumferential direction, or may be an azimuthal varying field (AVF) type magnetic field. For any type of magnetic field, the main magnetic field distribution is a non-isochronous magnetic field. The main magnetic field distribution is defined so as to satisfy the beam stabilization condition that the n value represented by (Equation 1) shown in FIG. 3 is larger than 0 and less than 1.

Here, ρ is the deflection radius of the design orbit, B is the magnetic field strength, and ∂B/∂r is the magnetic field gradient in the radial direction. Under the above-described beam stabilization condition, a beam slightly shifted in the radial direction from the design orbit receives a restoring force to return to the design orbit, and a beam shifted in a direction perpendicular to the orbital surface receives a restoring force from the main magnetic field in a direction to return to the orbital surface. That is, the beam causes the vicinity of the design orbit to be betatron oscillated so as to be stably circulated and accelerated. Furthermore, in the beam of total energy, the betatron oscillation frequency (horizontal tune) vr in the direction parallel to the orbital surface and orthogonal to the orbit is set to a value close to 1. The main magnetic field distribution described above is formed by the main magnetic pole 38 and a trim coil and a magnetic pole piece (both not illustrated) installed on the surface of the main magnetic pole 38. Since these constituent elements are arranged in a vertically symmetrical manner with respect to the orbital plane, the main magnetic field has only a magnetic field component in a direction perpendicular to the orbital plane on the orbital plane.

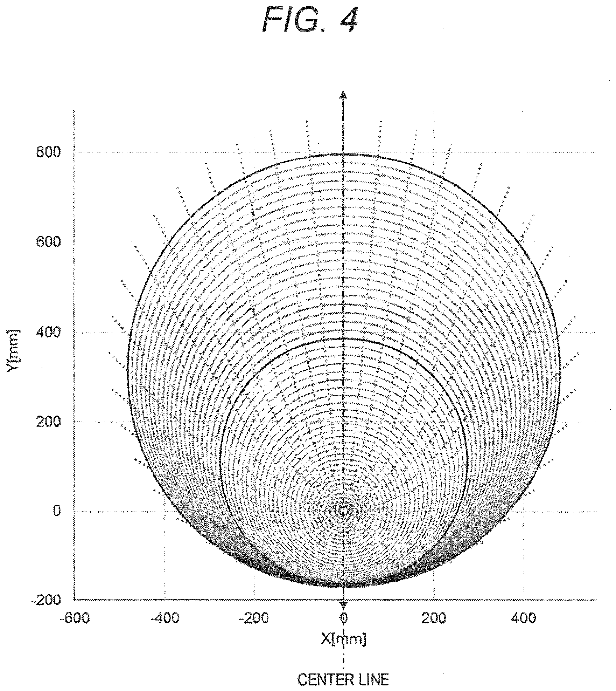

FIG. 4 illustrates the closed orbit of the beam having each energy. The upper side of FIG. 4 is the direction of the inner conductor 14, and the lower side of FIG. 4 is the direction of the entrance of beam extraction path 82.

In FIG. 4, orbits of 50 types of energy are indicated by solid lines at every magnetic rigidity modulus of 0.04 Tm from the maximum energy of 235 MeV. A dotted line is a line connecting the same circulating phase of each orbit, and is referred to as a synchronous phase line. The synchronous phase line is plotted for every circulating phase of π/20 from an aggregation region. The acceleration gap 11 formed between the dee electrode 12 and the dummy dee electrode 13 is installed along the synchronous phase line. More specifically, the dee electrode 12 has a hollow shape such as a fan shape having the vicinity of the center of the concentric orbit as the distal end and a radius lying along the synchronous phase line.

The orbit of the region where the energy of the beam is low is close to the concentric orbit having the vicinity of the ion injection portion 52 as the center, similarly to the known cyclotron. The orbit of the greater energy is densely aggregated on the entrance of beam extraction path 82 side. On the other hand, on the inner conductor 14 side, the orbits of the respective energies are in a positional relationship of being separated from each other. A point where the orbits are densely gathered is referred to as an aggregation region, and a discrete region is referred to as a discrete region. By forming such an orbit arrangement and extracting the beam from the vicinity of the aggregation region, a required beam kick amount can be reduced, so that beam extraction with variable energy can be facilitated.

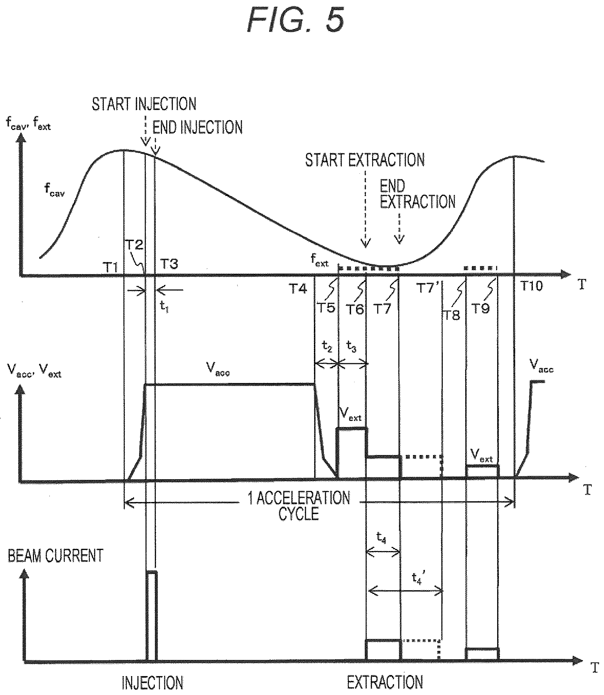

A process from when the beam is injected to the circular accelerator 39 until when the beam is extracted from the circular accelerator 39 will be described with reference to FIG. 5. On the upper side of FIG. 5, a graph showing a relationship between the resonance frequency fcav of the accelerating hollow 10, the frequency fext which is the frequency of the radio frequency electric field applied to the beam by the radio frequency kicker 70, and the time T is shown. At the center of FIG. 5, a graph showing a relationship between the acceleration voltage Vacc generated in the acceleration gap 11, the radio frequency voltage Vext applied to the radio frequency kicker 70, and the time T is shown. On the lower side of FIG. 5, a graph showing a relationship between the current of the injected beam and the current of the extracted beam and the time T is shown. Note that the radio frequency applied to the radio frequency kicker 70 corresponds to an example of a "second radio frequency".

One acceleration period starts from the rise of the acceleration voltage Vacc (time T1). Thereafter, when the acceleration voltage Vacc sufficiently increases, the beam is injected on the circular accelerator 39 from the ion source 53 (time T2). After an elapse of time t1 from the injection of the beam on the circular accelerator 39, the radio frequency capture of the beam ends. The captured beam, that is, the beam ready for acceleration among the injected beams starts to be accelerated by the acceleration voltage Vacc (time T3). When the energy of the beam reaches the energy to be extracted, the cutoff of the acceleration radio frequency is started (time T4), and when time t2 elapses therefrom, the acceleration voltage Vacc is turned OFF (time T5), and the beam circulates on a certain orbit.

Note that the individual charged particles forming the beam oscillate in a direction orthogonal to the orbit of the beam at the time of circulating, and this oscillation is referred to as a betatron oscillation, and the vibration frequency of the betatron oscillation is referred to as a betatron oscillation frequency. In addition, the oscillation frequency per circulating is referred to as tune, and the displacement on the r axis of the beam to the outer side of the orbital surface per circulating is referred to as turn separation. With respect to the circulating beam, the betatron oscillation in the direction orthogonal to the orbit of the beam in the orbit al surface is referred to as a horizontal betatron oscillation, and the tune is referred to as a horizontal tune. This betatron oscillation has a property that resonance occurs and amplitude rapidly increases when an appropriate radio frequency voltage is applied.

When the acceleration voltage Vacc is turned OFF, the application of the radio frequency voltage Vext to the radio frequency kicker 70 is started. Note that the start of the application of the radio frequency voltage Vext to the radio frequency kicker 70 (time T5) does not have to be exactly the same as when the acceleration voltage Vacc enters the OFF state. The start of application of the radio frequency voltage Vext may be immediately before, simultaneously with, or immediately after the start of cutoff of the acceleration radio frequency (time T4), or may be immediately before or immediately after the acceleration voltage Vacc is in the OFF state. Note that the energy to be extracted can be controlled by the application time of the acceleration voltage Vacc.

The radio frequency voltage of the radio frequency kicker 70 quickly rises with a response of several µs if the radio frequency kicker 70 does not have a resonator structure and is designed so that the capacitance has an appropriate value. The betatron oscillation has the property that the amplitude increases resonantly when the product of either the horizontal tune or the fractional part of the horizontal tune and the circulating frequency of the beam is substantially the same as the frequency of the applied radio frequency voltage. Therefore, the frequency fext of the radio frequency voltage is defined so as to be substantially the same as the product Δνr × frev of the fractional part Δνr of the horizontal tune vr of the maximum energy beam and the circulating frequency frev of the beam of energy to be extracted. Alternatively, a radio frequency voltage of a finite frequency bandwidth including frequency components that are substantially the same as the product Δνr × frev may be applied. As a result, the amplitude of the horizontal betatron oscillation continues to increase resonantly, and the beam eventually reaches a peeler magnetic field region 44 and a regenerator magnetic field region 45 installed on the outer circumferential side of the maximum energy orbit 80 (time T6).

The beam that has reached the peeler magnetic field region 44 is kicked to the outer peripheral side of the orbital surface. The beam that has reached the regenerator magnetic field region 45 is kicked to the inner peripheral side of the orbital surface. Kicking refers to deflecting a beam by applying an electric field or a magnetic field. The quadrupole magnetic field component of the peeler magnetic field region 44 causes the beam to be kicked further toward the outer peripheral side, and the turn separation increases. At the same time, the magnetic field of the regenerator magnetic field region 45 suppresses the horizontal tune of the beam from drastically fluctuating and prevents the betatron oscillation from diverging in the perpendicular direction orthogonal to the horizontal direction at 90 degrees and the beam from being lost before the beam is extracted. When the respective magnetic field strengths of the peeler magnetic field region 44 and the regenerator magnetic field region 45 are appropriately adjusted, resonance conditions of the betatron oscillation of 2vr = 2 can be generated to increase the turn separation.

As illustrated in FIG. 2, a septum coil 43 is installed at the entrance of beam extraction path 82. When the turn separation greatly exceeding the thickness of the coil conductor (not illustrated) installed on the inner peripheral side of the septum coil 43 is obtained, the beam is guided into the septum coil 43, sufficiently deflected, guided to the high energy beam transport system 47, and then extracted.

Note that immediately after the application of the radio frequency voltage to the radio frequency kicker 70 is started (time T5), the radio frequency voltage as large as possible is applied to quickly increase the amplitude of the beam, so that the time until the beam extraction can be shortened. The beam extraction current can be finely controlled by decreasing the radio frequency voltage immediately before the beam reaches the peeler magnetic field region 44 or the regenerator magnetic field region 45 (time T6) and adjusting the amount of the beam advancing to the peeler magnetic field region 44 and the regenerator magnetic field region 45. Instead of decreasing the radio frequency voltage Vext, the beam extraction current can be changed by sweeping the frequency of the radio frequency applied to the radio frequency kicker 70 or changing the phase of the gear radio frequency. This utilizes a property that the betatron oscillation frequency of charged particles included in the beam varies with a certain distribution (tune spread). The extraction current of the beam can be changed by changing the frequency of the radio frequency and changing the band of the distribution of the oscillation frequency of the charged particles that cause resonance.

When the application of the radio frequency voltage Vext to the radio frequency kicker 70 is stopped after elapse of time t4 from the start of the beam extraction (time T6), the beam extraction is stopped (time T7). The extraction time can be controlled by adjusting the time t4.

The beam extraction current can be adjusted by controlling the radio frequency voltage to apply to the radio frequency kicker 70, and the beam extraction can be stopped by stopping the application of the radio frequency voltage. Therefore, the spot dose required for scanning irradiation can be irradiated with one extraction pulse beam without excess or deficiency, and the dose rate is improved. For example, as illustrated in FIG. 5, if the application of the radio frequency voltage Vext to the radio frequency kicker 70 is continued until elapse of time t4' from the start of beam extraction (time T6), the beam can be extracted until time T7'.

If a beam circulating in the accelerator remains after extraction, the beam extraction can be resumed by applying the radio frequency voltage Vext again (time T8), and the beam can be used for the next spot irradiation without performing injection, capture, and acceleration of the beam again. That is, since the beam can be extracted a plurality of times within one acceleration period, the charge injected from the ion source 53 can be used without waste, and the dose rate is further improved. When the acceleration voltage Vacc starts to rise again, a new acceleration period starts (time T10).

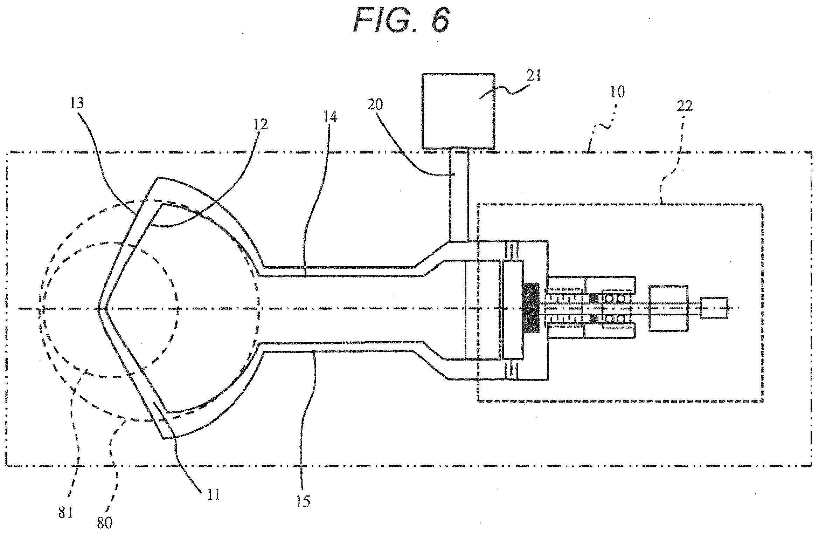

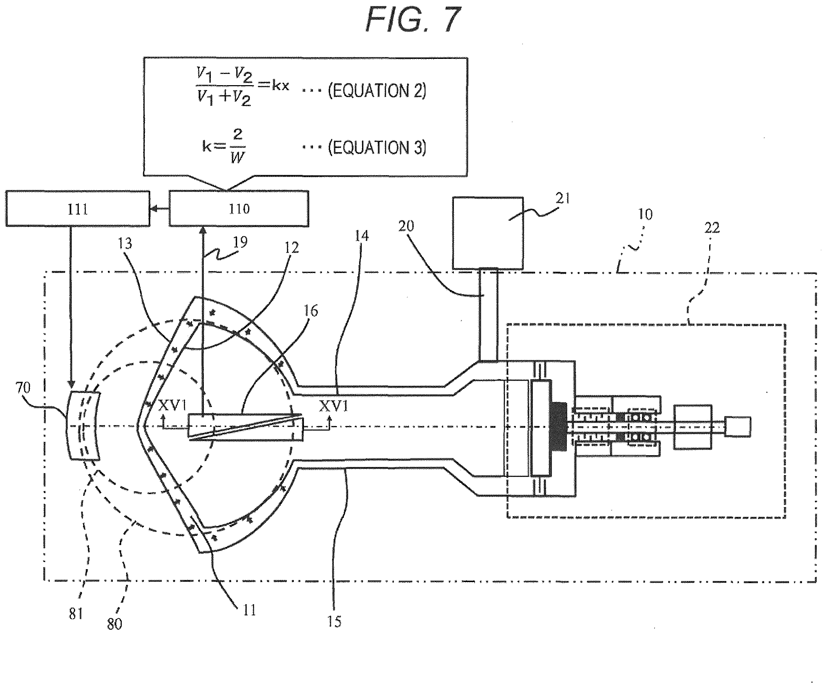

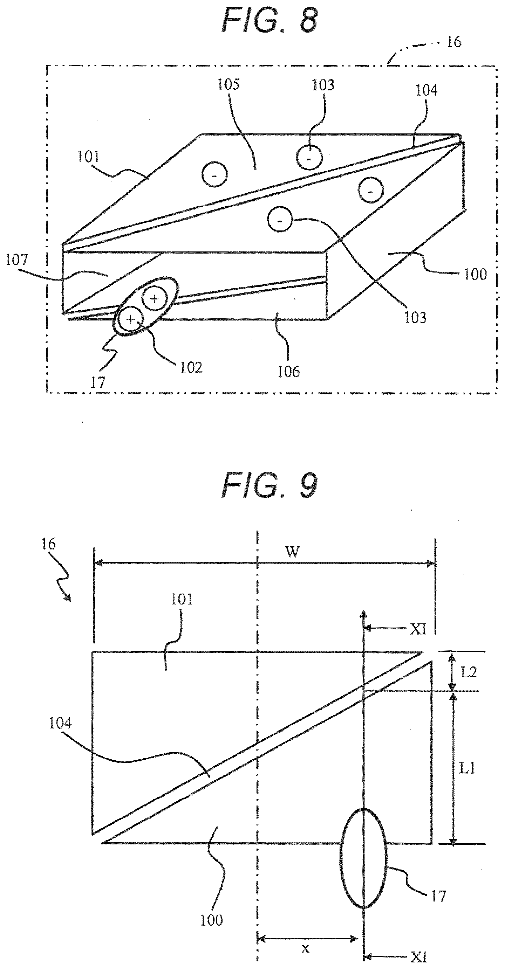

A configuration example of the circular accelerator 39 including the beam position monitor 16 according to the present embodiment will be described. FIG. 6 is a cross-sectional view illustrating the accelerating cavity 10 and the rotating capacitor 22. FIG. 7 is a cross-sectional view illustrating the accelerating cavity 10 and the beam position monitor 16. FIG. 8 is a schematic diagram of the beam position monitor 16 using a triangular plate electrode. FIG. 9 is a plan view of the beam position monitor 16.

As illustrated in FIG. 7, the circular accelerator 39 incorporates the beam position monitor 16. As an example of the beam position monitor 16, a beam position monitor in which U-shaped triangular plate electrodes 100,101 face each other such that their openings face each other will be described.

Although the principle of the beam position measurement using the triangular plate electrode will be described later, the beam position is calculated based on the potential that changes as the beam passes through. The beam position monitor 16 is disposed between the dee electrodes 12 of the accelerating cavity 10. The circular accelerator 39 of the present example has an eccentric orbit, and the beam position monitor 16 is provided so as to cover all the orbits from the maximum energy orbit 80 to the minimum extraction energy orbit 81 in order to monitor the position of the beam of all the energies to be extracted.

An acceleration electric field is generated in the acceleration gap 11. An example of the vector of the acceleration electric field is indicated by a plurality of small arrows. Since this acceleration electric field does not curve and enter between the dee electrodes 12, the electric field between the dee electrodes 12 becomes very weak. Among them, the region between the dee electrodes 12 on the rotating capacitor 22 side is the farthest from the acceleration gap 11, so that the acceleration electric field is minimized. In a case where the beam position monitor 16 includes two triangular plate electrodes facing each other, the position of the beam is calculated by the potential, and thus the detection accuracy of the beam position decreases when the acceleration electric field is strong. Therefore, in the present example, the beam position monitor 16 is provided at a position where the influence of the acceleration electric field is small. That is, the beam position monitor 16 is arranged in a region on the rotating capacitor 22 side between the dee electrodes 12. The electric field in the region where the beam position monitor 16 is arranged becomes smaller than the maximum electric field of the acceleration electric field (e.g., becomes 1/100,000 or less). 1/100,000 is an example for description and does not limit the scope of the present disclosure.

The voltage signal output from the beam position monitor 16 passes through the beam position monitor signal line 19 and is input to a beam position monitor signal processing unit 110 serving as an example of a "control circuit". The voltage signal is saved as position information in the beam position monitor. 16 from (Equation 2) and (Equation 3) in FIG. 7. That is, where the beam has passed through in the width dimension W of the beam position monitor 16 extending along the center line CL is detected and stored.

The beam position monitor signal processing unit 110 acquires and saves beam position information in time series. The beam position monitor signal processing unit 110 calculates the betatron oscillation frequency of the charged particle beam 17 from the time-series data of the beam position information. A calculation method will be described later.

Information on the calculated betatron oscillation frequency of the charged particle beam 17 is input from the beam position monitor signal processing unit 110 to the radio frequency kicker control unit 111.

The radio frequency kicker control unit 111 generates a control signal so as to generate a similar frequency of the RF electric field in the radio frequency kicker 70 based on the betatron oscillation frequency, and transmits a radio frequency output based on the generated control signal to the radio frequency kicker 70. This series of control feedback may be carried out during acceleration or after acceleration of the charged particle beam 17. In a case where the RF electric field has already been applied to the radio frequency kicker 70 without passing through the beam position monitor signal processing unit 110 in advance, overwriting with the frequency input from the beam position monitor signal processing unit 110 or control to approach the frequency by PID control may be performed in the radio frequency kicker control unit 111.

An operation principle of the beam position monitor 16 formed by the triangular plate electrodes 100,101 will be described with reference to FIGS. 8 and 9.

As illustrated in FIG. 8, the triangular plate electrodes 100,101 formed in a lateral U-shape are disposed to face each other such that their longest sides face each other. A slit 104 is formed by facing the respective long sides of the triangular plate electrodes 100,101 with each other with a gap interposed therebetween. An upper electrode and a lower electrode of the triangular plate electrode 100 are electrically conducted and have the same potential. Similarly, an upper electrode and a lower electrode of the triangular plate electrode 101 are electrically conducted and have the same potential.

In FIG. 8, the electrode on the near side of the plane of drawing is referred to as a right triangular plate electrode 100, and the electrode on the far side of the plane of drawing is referred to as a left triangular plate electrode 101. When the beam 17 passes through a cavity (beam passage) 107 formed by each electrode 100,101, the protons 102 in the beam 17 attract electrons 103 to the right triangular plate electrode 100 and the left triangular plate electrode 101, respectively. As a result, a potential difference is generated in the slit 104 of the right triangular plate electrode 100 and the left triangular plate electrode 101. The beam position can be calculated from this potential difference.

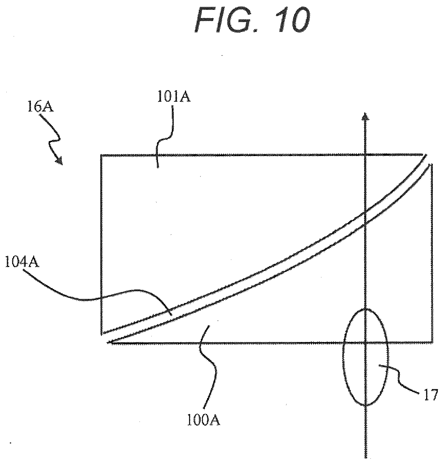

Note that in FIG. 8, the slit between the right triangular plate electrode 100 and the left triangular plate electrode 101 is linear, but this is not the sole case. For example, as in a beam position monitor 16A illustrated in FIG. 10, a slit 104A formed between triangular plate electrodes 100A and 101A may include a curve in at least a part thereof.

A method of calculating the beam position will be described with reference to FIG. 9. Of the right triangular plate electrode 100 and the left triangular plate electrode 101, an electrode length orthogonal to the injection direction of the beam 17 on the same plane is defined as an electrode length W. In FIG. 9, the center line of the electrode length W is represented by a one-dot chain line, and the distance from the one-dot chain line to the centroid position of the beam 17 is set as x. When the advancing direction of the beam 17 is displaced in the left-right direction in FIG. 9 between the right triangular plate electrode 100 and the left triangular plate electrode 101, charges are excited in proportion to the length of the beam passing through each triangular plate electrode 100,101. That is, assuming that the voltage excited by the right triangular plate electrode 100 is V1 and the voltage excited by the left triangular plate electrode 101 is V2, the shift x of the passing position of the beam from the center in the width direction of each triangular plate electrode 100,101 can be obtained by (Equation 2) and (Equation 3) illustrated in FIG. 7.

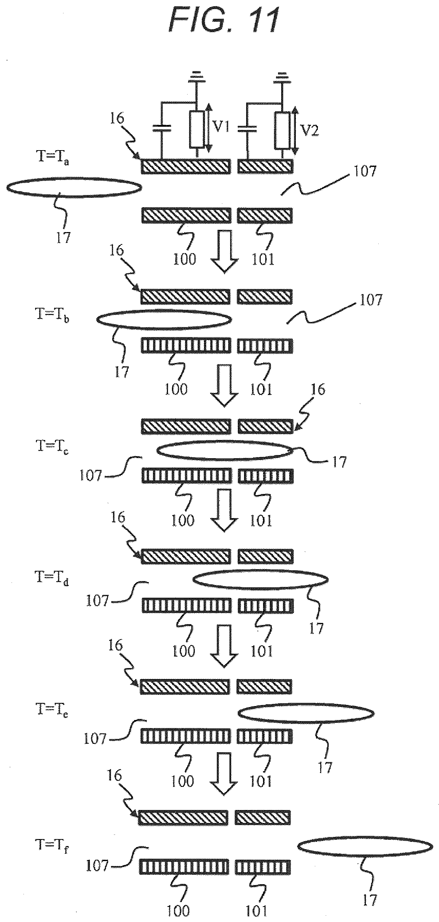

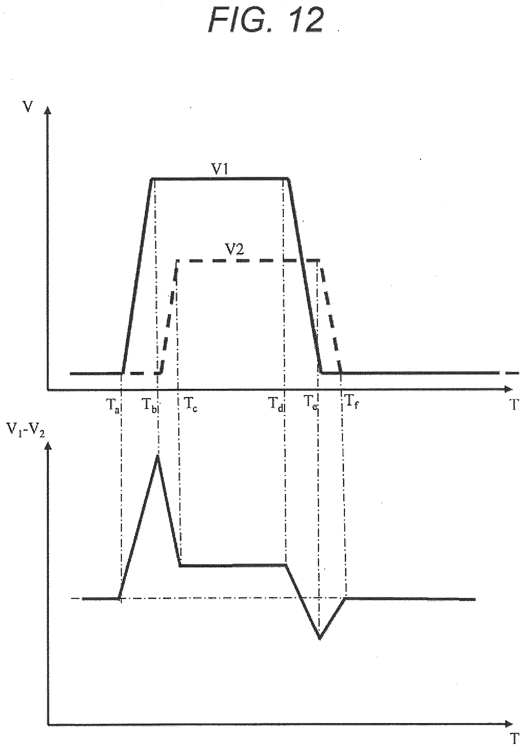

A temporal change of the voltage output from the beam position monitor 16 will be described with reference to FIGS. 11 and 12. FIG. 11 is a cross-sectional view as viewed from X1-XI direction in FIG. 9. Therefore, in FIG. 11, the right-side electrode 100 is shown on the left side, and the left-side electrode 101 is shown on the right side. At time Ta illustrated at the top of FIG. 11, the beam 17 is deviated from the beam position monitor 16, and thus no voltage is excited in the right triangular plate electrode 100 and the left triangular plate electrode 101.

At time Tb, the beam 17 enters the right triangular plate electrode 100, and the voltage V1 is excited. At time Tc, the beam 17 enters the right triangular plate electrode 100 and the left triangular plate electrode 101, and thus the voltage V1 and the voltage V2 are excited. From time Tc to time Td, the beam 17 enters across the right triangular plate electrode 100 and the left triangular plate electrode 101, and thus the voltage V1 and the voltage V2 are excited at constant voltages.

At time Te, the beam 17 passes the right triangular plate electrode 100 and passes through only the left triangular plate electrode 101, and thus only the voltage V2 is excited. At time Tf, the beam 17 passes both the right triangular plate electrode 100 and the left triangular plate electrode 101, and thus the voltages are not excited. That is, the beam 17 is deviated from the beam position monitor 16. Here, according to (Equation 2) and (Equation 3), since a signal for a time during which the voltage V1 and the voltage V2 become constant values is required, the detection accuracy of the beam position degrades unless the signal from time Tc to time Td are used in a discriminated manner.

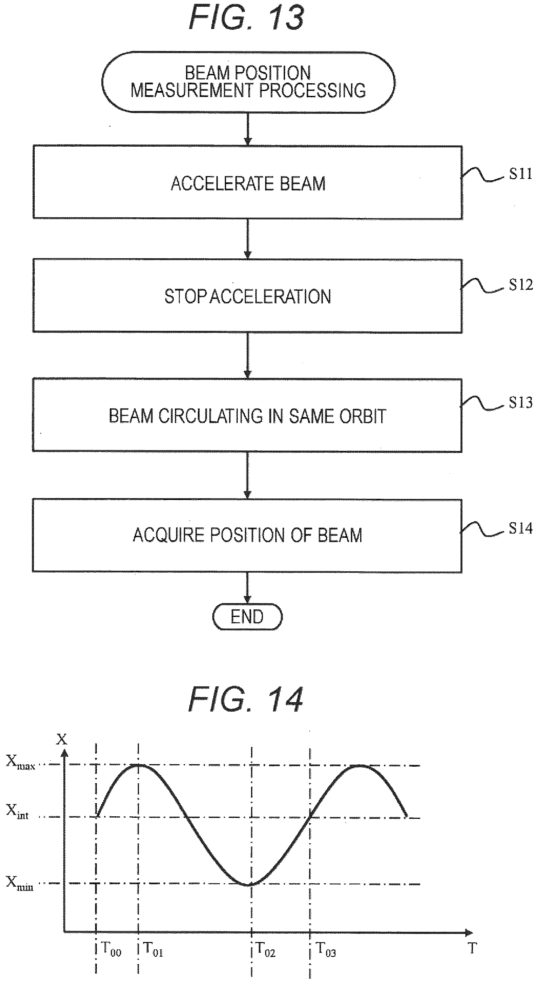

A method of calculating the betatron oscillation frequency of the charged particle beam 17 from the beam position measured by the beam position monitor 16 will be described with reference to FIGS. 13 to 15.

In step S11, the circular accelerator 39 accelerates the charged particle beam 17 to the energy to be extracted. In step S12, the circular accelerator 39 stops the supply of the RF power from the radio frequency power supply 21, and turns OFF the acceleration voltage applied to the acceleration void 11. In step S13, the charged particle beam 17 that is no longer accelerated circulates an orbit having the same radius of curvature. In step S14, the position of the charged particle beam 17 at the time of circulating is acquired in time series.

An example of the acquired data is illustrated in FIG. 14. Since the betatron oscillation frequency is lower than the circulating frequency of the charged particle beam 17, the betatron oscillation frequency is output in a sine wave shape. As an example, the betatron oscillation frequency is several hundred to several 1/10 with respect to the circulating frequency, and sufficient sampling can be performed to acquire sine wave data.

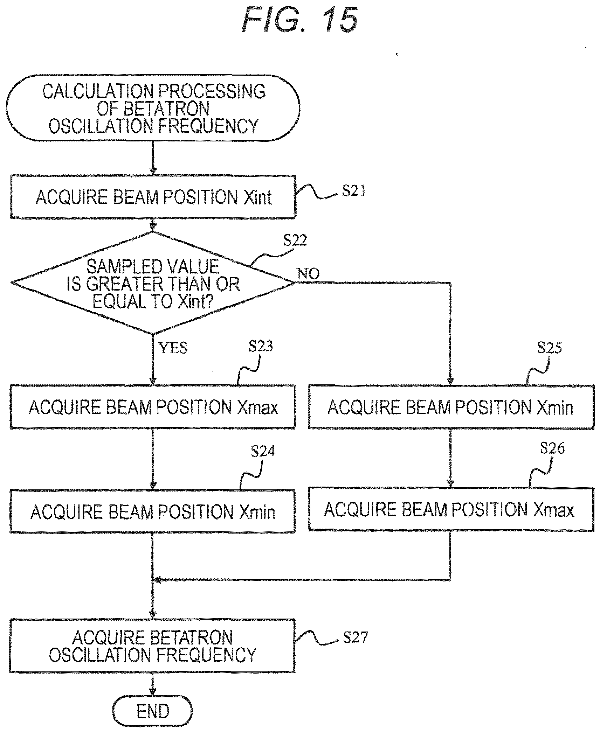

Furthermore, a method of calculating the betatron oscillation frequency by the beam position monitor 16 will be described with reference to FIGS. 14 and 15. The time at which the beam position monitor 16 starts to acquire the position of the charged particle beam 17 is defined as T00, and the position of the charged particle beam 17 calculated at that time is defined as an initial value Xint. The position of the charged particle beam 17 at time T01 is defined as a maximum Xmax. The position of the charged particle beam 17 at time T02 is defined as a minimum Xmin. The position of the charged particle beam 17 at time T03 is returned to the initial value Xint again.

In step S21, an initial value (initial position) Xint at time T00 is acquired. In step S22, the sampled value (sampling data) is compared with the initial value Xint. When the sampling data is greater than or equal to the initial value Xint (S22: YES), a flow of searching for the maximum value Xmax is carried out (S23, S24). In step S23, the positions X at the preceding and subsequent times are compared, and the maximum value Xmax is acquired from a gradient method of a differential value. In step S24, the values of the positions X at the preceding and subsequent times after time T01 are compared, and the minimum value Xmin is acquired from the gradient method of the differential value. Thereafter, the processing proceeds to step S27.

On the other hand, in step S22, when the value of the sample data next to time T00 is smaller than the initial value Xint (S22: NO), a flow of searching for the minimum value Xmin is carried out (S25, S26). In step S25, the values of the positions X at the preceding and subsequent times are compared, and the minimum value Xmin is acquired from the gradient method of the differential value. In step S26, the values of the positions X at the preceding and subsequent times after time T02 are compared, and the maximum value Xmax is acquired from the gradient method of the differential value. Thereafter, the processing proceeds to step S27.

In step S27, twice the time T01 to 02 between time T01 and time T02 is substituted as a period T of the betatron oscillation. A reciprocal of the period T is the betatron oscillation frequency.

As another method, from the time series data at the position X, the data at the time of the maximum value may be set as the maximum value Xmax, and the data at the time of the minimum value may be set as the minimum value Xmin. As still another method, a delay of a short time may be provided after the initial value Xint is acquired at time T00, and when data falls within an error range of a certain extent of the initial value Xint after the delay, the betatron oscillation frequency may be acquired with the time until then as one period.

Here, a horizontal tune may be used instead of the betatron oscillation frequency input from the beam position monitor signal processing unit 110 to the radio frequency kicker control unit 111, in which case, Fourier expansion is performed on that from which a signal of when the beam has passed through is extracted by the beam position monitor 16 to calculate the horizontal tune.

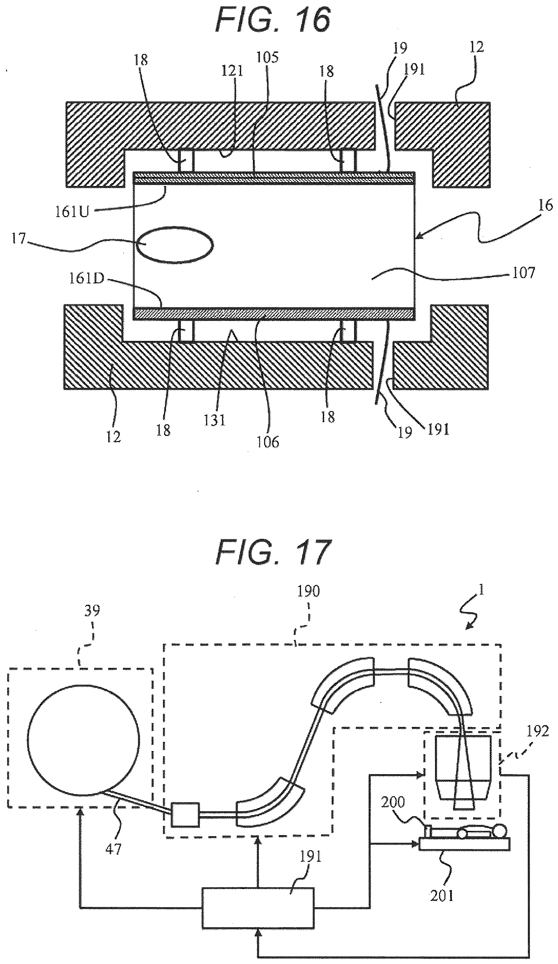

FIG. 16 illustrates an example of the beam position monitor 16 disposed in the dee electrode 12. FIG. 16 is a cross-sectional view as viewed from the XVI-XVI direction in FIG. 7. Grooves 121,131 for disposing the beam position monitor 16 are formed in the dee electrode 12. The groove 121,131 is an example of an "attachment portion".

The beam position monitor 16 is supported in the grooves 121,131 using an insulating spacer 18 serving as an example of an "insulation member" in order to respectively maintain electrical insulation between the dee electrodes 12.

When the dee electrode 12 is configured by the right triangular plate electrode 100 and the left triangular plate electrode 101, an acute angle portion of the triangular plate becomes thin and easily bends. Therefore, more insulating spacers 18 may be disposed than other portions at the acute angle portion of the triangle. Alternatively, a more rigid insulating spacer may be used. Since the electrode 105 of the beam position monitor 16 at the upper part has its own weight, it is made to excel more in withstanding load than the insulating spacer 18 that supports the electrode 106 at the lower part.

Opposing surfaces 161U and 161D of the beam position monitor 16 may be located on substantially the same plane as the lower surface of the dee electrode 12 and the upper surface of the dee electrode 12. That is, in FIG. 16, the lower surface 161U of the upper electrode 105 of the beam position monitor 16 and the lower surface of the dee electrode 12 may be located on substantially the same plane, and the upper surface 161D of the lower electrode 106 of the beam position monitor 16 and the upper surface of the dee electrode 12 may be located on substantially the same plane. In this way, the corner portion of the conductor exposed to a space 107 between the dee electrodes 12 is reduced, and the discharge probability can be reduced.

A through hole 191 through which the beam position monitor signal line 19 passes is formed in each of the dee electrodes 12. At least one beam position monitor signal line 19 is connected to the beam position monitor 16 through the through hole 191. The beam position monitor signal line 19 may be a conducting wire, a coaxial cable, or the like. The through hole 191 for the beam position monitor signal line 19 may be formed in a surface facing the dee electrode 12 and the beam position monitor 16. In this way, the beam position monitor 16 and the dee electrode 12 can serve as capacitance, and the leakage of the radio frequency (RF) to the outside of the accelerating cavity 10 can be suppressed.

According to the present example configured as described above, the charged particle beam circulating in the orbit having different radii of curvature according to the energy can be non-destructively measured during the operation of the circular accelerator 39.

Furthermore, in the present example, since the betatron oscillation frequency calculated from the detection signal of the beam position monitor is synchronized with the radio frequency electric field of the kicker, the beam extraction efficiency (extraction efficiency) by the kicker can be improved.

### Second Example

A second example will be described with reference to FIG. 17. In the present example, a particle beam therapy system 1 including the circular accelerator 39 described in the first example will be described.

The particle beam therapy system 1 illustrated in FIG. 17 includes, for example, a circular accelerator 39, a rotary gantry 190, an irradiation device 192 including a scanning coil, a treatment couch 201, and a control device 191 configured to control these components.

A beam extracted from the circular accelerator 39 is transported to the irradiation device 192 by the rotary gantry 190. The transported ion beam is shaped so as to match the shape of the affected area by the irradiation device 192 and the adjustment of the beam energy, and the target of the affected area of a patient 200 lying on the treatment couch 201 is irradiated with a predetermined amount. The irradiation device 192 includes a dose monitor (not illustrated) and monitors the dose emitted on the patient 200 for each irradiation spot. The control device 191 calculates a required dose to each irradiation spot based on the dose data, and outputs the calculation result to the circular accelerator 39.

According to the present example configured as described above, the throughput of the patient receiving radiotherapy treatment can be increased.

Note that the present invention is not limited to the above-described embodiment. Those skilled in the art can make various additions and modifications within the scope of the present invention. In the above-described embodiment, the present invention is not limited to the configuration example illustrated in the accompanying drawings. The configuration and the processing method of the embodiment can be appropriately modified within the scope of achieving the object of the present invention.

In addition, each constituent element of the present invention can be arbitrarily picked and selected, and an invention having the picked and selected configuration is also included in the present invention. Furthermore, the configurations described in the claims can be combined with other than the combinations clearly described in the claims.

For example, the present embodiment can be considered to include the following configurations.

(Configuration 1) A circular accelerator configured to apply a first radio frequency to a circulating charged particle beam to accelerate the charged particle beam, the circular accelerator including: an electrode configured to apply the first radio frequency to the charged particle beam; and a beam position monitor provided in the electrode and configured to detect a position of the charged particle beam.

(Configuration 2) The circular accelerator according to configuration 1, in which the beam position monitor is an electrostatic coupling beam position monitor.

(Configuration 3) The circular accelerator according to configuration 1 or 2, in which a beam closed orbit in which the charged particle beam circulates by a static magnetic field is formed, and the charged particle beam is accelerated by modulating the first radio frequency.

(Configuration 4) The circular accelerator of any one of configurations 1 to 3, in which the electrode is a dee electrode.

(Configuration 5) The circular accelerator of any one of configurations 1 to 4, in which the beam position monitor is a triangular plate electrode.

(Configuration 6) The circular accelerator according to any one of configurations 1 to 5, in which the beam position monitor is attached to an attachment portion provided in the dee electrode by way of an insulation member.

(Configuration 7) The circular accelerator according to any one of configurations 1 to 6, in which the attachment portion is a groove provided in the dee electrode, and the beam position monitor is attached to the groove such that an electrode surface of the beam position monitor is flush with an electrode surface of the dee electrode.

(Configuration 8) The circular accelerator according to any one of configurations 1 to 7, in which the beam position monitor is electrically connected to a control circuit via a signal line penetrating the electrode, and the control circuit calculates a position of the charged particle beam based on a signal acquired from the beam position monitor via the signal line.

(Configuration 9) The circular accelerator according to any one of configurations 1 to 8, in which a second radio frequency applied to a radio frequency kicker that extracts the charged particle beam is controlled based on the position of the charged particle beam detected by the beam position monitor.

(Configuration 10) The circular accelerator of any one of configurations 1 to 9, in which a betatron oscillation frequency of the charged particle beam is obtained based on the position of the charged particle beam detected by the beam position monitor, and a frequency of the second radio frequency is controlled based on the betatron oscillation frequency.

(Configuration 11) A particle beam therapy system comprising: the circular accelerator according to any one of configurations 1 to 10; and an irradiation device configured to irradiate a patient with the charged particle beam extracted from the circular accelerator.

### Reference Signs List

- 1: particle beam therapy system
- 10: accelerating cavity
- 11: acceleration gap
- 12: dee electrode
- 13: dummy dee electrode
- 14: inner conductor
- 15: outer conductor
- 16: beam position monitor
- 17: charged particle beam
- 18: insulating spacer
- 19: beam position monitor signal line
- 20: input coupler
- 21: radio frequency power supply
- 22: rotating capacitor
- 38: main magnetic pole
- 39: circular accelerator
- 40: main electromagnet
- 41: yoke
- 42: main coil
- 43: septum coil
- 44: peeler magnetic field region
- 45: regenerator magnetic field region
- 46: beam through hole
- 47: high energy beam transport system
- 48: coil through hole
- 49: vacuum drawing through hole
- 50: radio frequency system through hole
- 52: ion injection portion
- 53: ion source
- 54: low energy beam transport system
- 60: cryostat
- 70: radio frequency kicker
- 80: maximum energy orbit
- 81: minimum extraction energy orbit
- 82: entrance of beam extraction path
- 86: radio frequency kicker power supply
- 100: right triangular plate electrode
- 101: left triangular plate electrode
- 102: proton
- 103: electron
- 104: slit
- 105: upper electrode
- 106: lower electrode
- 107: passage through which beam passes
- 110: beam position monitor signal processing unit
- 111: radio frequency kicker control unit
- 121, 131: groove
- 190: rotary gantry
- 191: control device
- 192: irradiation device
- 200: patient
- 201: treatment couch

## Claims

1. A circular accelerator configured to apply a first radio frequency to a circulating charged particle beam to accelerate the charged particle beam, the circular accelerator comprising:
an electrode configured to apply the first radio frequency to the charged particle beam; and
a beam position monitor provided in the electrode and configured to detect a position of the charged particle beam.

2. The circular accelerator according to claim 1, wherein the beam position monitor is an electrostatic coupling beam position monitor.

3. The circular accelerator according to claim 2, wherein
a beam closed orbit in which the charged particle beam circulates by a static magnetic field is formed, and
the charged particle beam is accelerated by modulating the first radio frequency.

4. The circular accelerator of any one of claims 1 to 3, wherein the electrode is a dee electrode.

5. The circular accelerator of any one of claims 1 to 3, wherein the beam position monitor is a triangular plate electrode.

6. The circular accelerator according to claim 4, wherein the beam position monitor is attached to an attachment portion provided in the dee electrode by way of an insulation member.

7. The circular accelerator according to claim 6, wherein
the attachment portion is a groove provided in the dee electrode, and
the beam position monitor is attached to the groove such that an electrode surface of the beam position monitor is flush with an electrode surface of the dee electrode.

8. The circular accelerator according to any one of claims 1 to 3, wherein
the beam position monitor is electrically connected to a control circuit via a signal line penetrating the electrode, and
the control circuit calculates a position of the charged particle beam based on a signal acquired from the beam position monitor via the signal line.

9. The circular accelerator according to any one of claims 1 to 3, wherein a second radio frequency applied to a radio frequency kicker that extracts the charged particle beam is controlled based on the position of the charged particle beam detected by the beam position monitor.

10. The circular accelerator of any one of claims 1 to 3, wherein
a betatron oscillation frequency of the charged particle beam is obtained based on the position of the charged particle beam detected by the beam position monitor, and
a frequency of the second radio frequency is controlled based on the betatron oscillation frequency.

11. A particle beam therapy system comprising:
the circular accelerator according to any one of claims 1 to 3; and
an irradiation device configured to irradiate a patient with the charged particle beam extracted from the circular accelerator.
